# EUROPEAN PATENT SPECIFICATION

(11) **EP 2 436 670 B1**
(45) Date of publication and mention of the grant of the patent: **16.04.2014**
(21) Application number: 10780053.4
(22) Date of filing: 24.05.2010
(51) Int. Cl.: A61K 31/4412, A61P 43/00, C07D 213/64

(54) **Preparation of 1-(substituted aryl)-5-trifluoromethyl-2-(1H)pyridone compounds and salts thereof and their applications**
HERSTELLUNG VON 1-(SUBSTITUIERTEN ARYL)-5-TRIFLUOROMETHYL-2-(1H)-PYRIDON-VERBINDUNGEN UND IHRER SALZE UND IHRE VERWENDUNGEN
PRÉPARATION DE COMPOSÉS DE 1-(ARYL SUBSTITUÉ)-5-TRIFLUOROMÉTHYL-2-(1H)-PYRIDONE ET DE LEURS SELS ET LEURS APPLICATIONS

(30) Priority: 25.05.2009 CN 200910043501
(43) Date of publication of application: 04.04.2012
(73) Proprietor: Central South University, Hunan 410013 (CN)
(72) Inventor: HU, Gaoyun, Changsha Hunan 410013 (CN); TAO, Lijian, Changsha Hunan 410013 (CN); CHEN, Jun, Changsha Hunan 410013 (CN)
(74) Representative: Bassil, Nicholas Charles
(86) International application number: PCT/CN2010/073105
(87) International publication number: WO 2010/135972

(56) References cited:
- WO-A1-2009/149188
- WO-A2-2006/122154
- CN-A- 101 235 030

## Description

### TECHNICAL FIELD

The invention relates to 1-(substituted aryl)-5-trifluoromethyl-2-(1H)pyridone compounds, preparation methods and medical applications for the same.

### BACKGROUND OF THE INVENTION

Fibrosis is in a variety of organs or tissues to cause reduction of parenchyma cells therein and increase of fibrous connective tissues, while eventually bringing damage of tissue structures, dysfunction or even organ failure. Mechanism, diagnostic methods and prevention measures for fibrosis of organs or tissues have been widely studied. In prior art, a considerable progress is made in some aspects, but some key issues unresolved still exist.

US patents US3839346A, US4052509A, US4042699 disclose 29 pyridone compounds having formula I as follows, 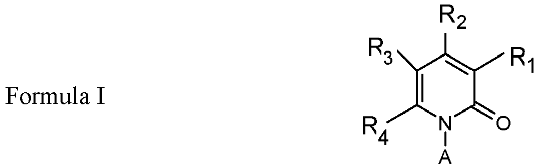 and disclose functions of the pyridone compounds of resisting inflammation, allaying fever, reducing level of serum uric acid, relieving pain or the like, wherein 5-methyl-1-phenyl-2(1H)-pyridone (Pirfenidone) has a best activity and a lower toxicity.

US patent US5,310,562 discloses 5-methyl-1-phenyl-2(1H)-pyridone for the first time in 1994, that is Pirfenidone (PFD), having an anti-fibrosis biological activity; subsequently US patent US5,518,729 and US5,716,632 disclose N-substituted-2-(1H)pyridone described as the structural formula I and N-substituted-3-(1H)pyridone having the same anti-fibrosis function. 44 compounds are specified, most of which are known compounds derived from US patent US4052509; and in the compounds, R1, R2, R3, and R4 are defined as methyl group or ethyl group.

Pirfenidone (PFD) is proven to have effectiveness in fibrosis prevention through in vitro and animal experiments. The pirfenidone has functions of stopping or even converting ECM accumulation and preventing or reversing fibrosis and scar formation in the experiments of animals with renal fibrosis and pulmonary fibrosis and in the clinical treatment of patients with idiopathic pulmonary fibrosis. (Shimizu T, Fukagawa M, Kuroda T, et al. Pirfenidone prevents collagen accumulation in the remnant kidney in rats with partial nephrectomy. Kidney Int, 1997,52 (Suppl 63): S239-243; Raghu G, Johnson WC, Lockhart D, et al. Treatment of idiopathic pulmonary fibrosis with a new antifibrotic agent, pirfenidone. Am J Respir Crit Care Med, 1999,159 : 1061-1069).

The applicant proposes a CN patent ZL02114190.8 and provides a class of pyridone compounds shown in the formula II. 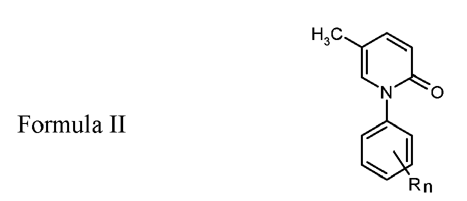
if n=1, substituent R is F, Br, or I;
if n=2, substituent R is F, Cl, Br, I, saturated linear alkyl group, oxo-saturated linear alkyl group, or halo-saturated linear alkyl group.

The substituent R is either at ortho-position, meta-position, para-position or the like on a benzene ring.

Pirfenidone has come into the market in Japan in 2008 for treating indications for pulmonary fibrosis, however Pirfenidone and its derivatives have strength not high enough. The clinical dose of Pirfenidone achieves 2400mg/day.

Patents WO2007053685 and WO2006122154 disclose compounds having functions of inhibiting p38 kinase, applied to treatment of fibrosis diseases and figured in the formula III; 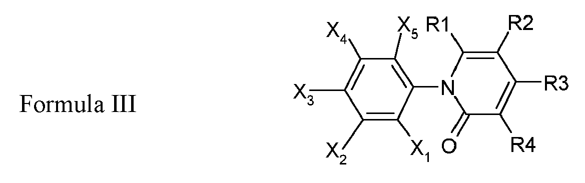 wherein, R1-R4 each are H, alkyl group, substituted alkyl group, alkenyl group, haloalkyl group, nitro alkyl group, hydroxyalkyl group, alkoxyl group, phenyl group, substituted phenyl group, halogen, hydroxyl group, alkoxyalkyl group, carboxyl group, alkoxycarbonyl group, etc.; X1-X5 each are H, halogen, alkoxyl group, or hydroxyl group.

WO2007062167 also discloses compounds having functions of inhibiting p38 kinase and applied to treatment of various fibrosis diseases, wherein some structures are shown as follows:

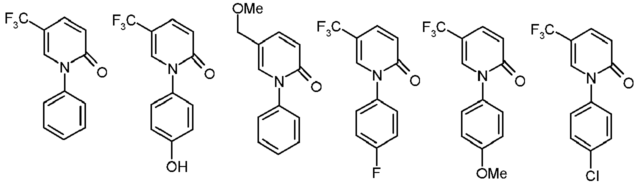

Some simple substituents are provided on the benzene rings of the compounds. CN patent 200710034357 discloses some similar compounds having the above structures with anti-fibrosis activity and a compound with the anti-fibrosis activity shown in the formula IV

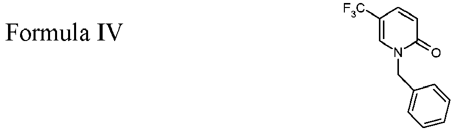

Those compounds are provided with TFM at 5-position of the pyridone ring, thereby overcoming the disadvantages of inferior action of Pirfenidone, but the strength of those compounds is still not powerful enough.

DE patent DE4343528 reports a class of compounds having insecticidal actions in agriculture, with the formula V as follows. 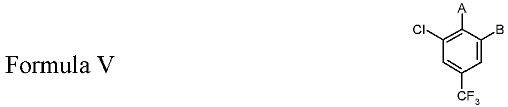 in structural formula V, A and B are substituted by various heterocyclic rings, such as furan ring, imidazole, pyridine and pyridone; wherein a class of compounds with the formula VI is included.

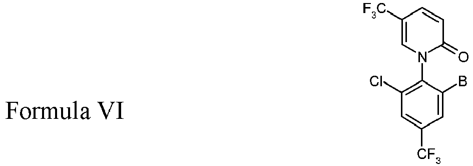

EP patents EP259048, EP367410 and EP398499 report a class of compounds having insecticidal actions in agriculture, with the formula VII as follows: 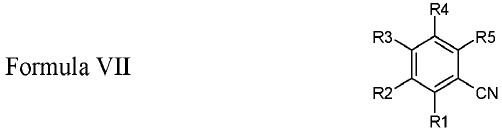 wherein a class of compounds having the formula VIII, in which R1 is pyridone and R10 is O or S, is included.

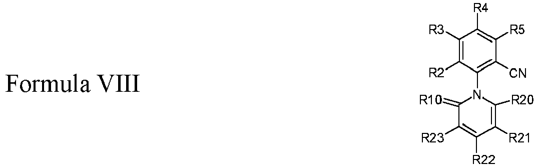

EP patent EP216541 reports a class of compounds having insecticidal actions in agriculture, with the formula IX as follows: 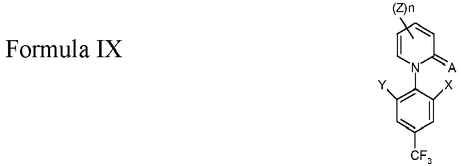 wherein a class of compounds with the formula X is included.

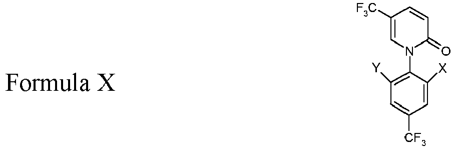

EP patent EP488220 reports a class of compounds having insecticidal actions, with the

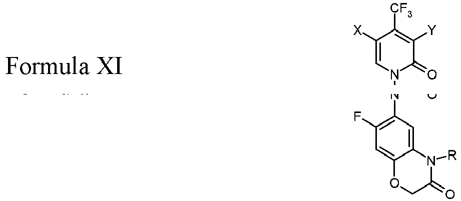

In structures of the above-mentioned compounds, the pyridine ring and the benzene ring at 1-position of the pyridine ring have a plurality of substituents; the compounds with complicated structures have not been reported to have the anti-fibrosis function. DE102004027359 discloses a class of compounds capable of adjusting dopamine-3 receptor and applied to treatment of Parkinson's disease and schizophrenosis; 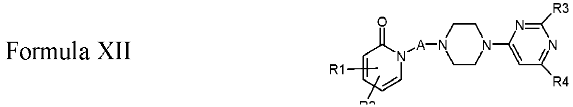 wherein, A is a hydrocarbon chain with 4-6 atoms, having 1-2 substituted methyl groups thereon; or 1-2 carbon atoms in the carbon chain are substituted by O, C=O, S and other atoms; R1 and R2 are H, CN, NO₂ halogen atom, OR⁵, NR⁶R⁷, C(O)*_{N}*R⁶R⁷, O-C(O)*_{N}*R⁶R⁷; C₁-C₆ alkyl group, C₁-C₆ haloalkyl group, etc.

CN101235030 describes 1-substituted -5-trifluoromethyl-2-(1H)pyridone compounds with anti-fibrosis function.

Accordingly, compounds in the conventional invention have low anti-fibrosis activities and strong liposolubility as a plurality of fluorine atoms are introduced onto molecules, thereby, the compounds can not be made into solutions because there is no highly water-soluble group in the molecule.

### SUMMARY OF THE INVENTION

The invention provides 1-(substituted phenyl)-5-trifluoromethyl-2-(1H)pyridine compounds shown in formula XIII, 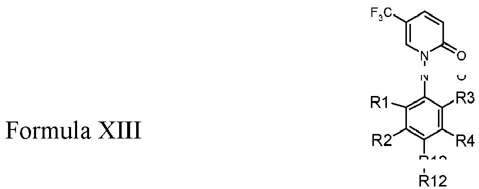 wherein R1-R4, R12 are selected from H, CN, NO₂, hydroxyl group, amino group, halogen atom, C₁-C₆ alkoxyl group, NR¹⁰R¹¹, C(O)R¹⁴, C₁-C₆ alkyl group, C₁-C₆ haloalkyl group, C₂-C₆ alkenyl group, carboxyl group and carboxylic ether; wherein R¹⁴ is C₁-C₆ alkyl group, R¹⁰ and R¹¹ are selected from H, C₁-C₆ hydroxyalkyl group, esterified C₁-C₆ hydroxyalkyl group, C₁-C₆ alkoxyalkyl group, or formula XIV;
and at least one of R1-R4 and R12 is NR¹⁰R¹¹ and at least one of R¹⁰ and R¹¹ is of the Formula XIV: 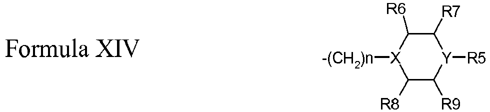 wherein R5 is selected from H, C₁-C₆ alkyl group, C₁-C₆ hydroxyalkyl group, esterified C₁-C₆ hydroxyalkyl group, and C₂-C₆ alkenyl group; R6-R9 are selected from H, C₁-C₆ alkoxyl group, =O, C₁-C₄ alkyl group, C₁-C₄ haloalkyl group, C₁-C₄ hydroxyalkyl group, and C₂-C₄ alkenyl group; X is selected from N and CH₂; Y is selected from N, O, and C with the proviso that when Y is O, R⁵ is absent; n is 1-6;
and pharmaceutically acceptable salts thereof.

More preferably, R12 is NR¹⁰R¹¹.

According to embodiments of the invention, more preferably, one of R1-R4 is a halogen atom and others are H if R12 is NR¹⁰R¹¹.

According to embodiments of the invention, the following compounds are preferred:
1-((2-chloro-4-((3-(4-methylpiperazin-1-yl)propyl)amino)phenyl)-5-(trifluoromethyl)pyridin-2 (1H)-one (compound 1);
1-((2-chloro-4-((3-morpholinylpropyl)amino)phenyl)-5-(trifluoromethyl)pyridin-2(1H)-one (compound 2);
1-((2-chloro-4-((3-piperidin-1-yl)propylamino)phenyl)-5-(trifluoromethyl)pyridin-2(1H)-one (compound 3);
1-((2-chloro-4-(((3-piperidin-1-yl)propyl)amino)phenyl)-5-(trifluoromethyl)pyridin-2(1H)-one hydrochloride (compound 7);
1-(((4-((piperazin-1-yl)ethyl)amino)-2-chlorophenyl)-5-(trifluoromethyl)pyridin-2(1H)-one (compound 9);
1-((2-chloro-4-((2-(piperidyl-1-yl)ethyl)amino)phenyl)-5-(trifluoromethyl)pyridin-2(1H)-one (compound 10);
1-((2-chloro-4-((2-morpholinylethyl)amino)-phenyl)-5-(trifluoromethyl)pyridin-2(1H)-one (compound 11);
1-((2-chloro-4-((2-(4-methylpiperazin-1-yl)ethyl)amino)phenyl)-5-(trifluoromethyl)pyridin-2( 1H)-one (compound 12);
1-((2-chloro-4-((2-(4-(2-hydroxyethyl)piperazin-1-yl)ethyl)amino)-phenyl)-5-(trifluoromethyl) pyridin-2(1H)-one (compound 13).

Pharmaceutically acceptable salts of the compounds of the invention can be hydrochlorate, sulfate, phosphate, perchlorate, methanesulfonate, trifluoromethanesulfonate, formate, acetate, propionate, butyrate, maleate, succinate, trifluoroacetate, succinate, salicylate, DL-aspartate, D-aspartate, L-aspartate, DL-glutamate, D-glutamate, L-glutamate, glycerate, succinate, stearate, DL-tartrate, D-tartrate, L-tartrate, (+/-)-mandelate, (R)-(-)-mandelate, (S)-(+)-mandelate, citrate, mucate, maleate, malonate, benzoate, DL-malate, D-malate, L-malate, hemimalate, 1-adamantane acetate, 1-adamantane carboxylate, flavianate, sulfoacetate, (+/-)-lactate, L-(+)-lactate, D-(-)-lactate, pamoate, D-α-galacturonic acid salt, glycerate, DL-cystine salt, D-cystine salt, L-cystine salt, DL-homocystine salt, D-homocystine salt, L-homocystine salt, DL-cysteine salt, D-cysteine salt, L-cysteine salt, (4S)-hydroxy-L-proline, cyclopropane-1,1-dicarboxylate, 2,2-methyl malonate, tyrosine salt, proline salt, fumarate, 1-hydroxy-2-naphthoate, phosphonoacetate, carbonate, bicarbonate, 3-phosphonopropionate, DL-pyroglutamate, D-pyroglutamate, L-pyroglutamate, toluenesulfonate, benzenesulfonate, esilate, (+/-)-camsilate, naphthalenesulfenesulfonate, 1R-(-)-camsilate,1S-(+)-camsilate, 1,5-napadisilate, 1,2-ethanedisulphonate, 1,3-propanedisulphonate, 3-(N-morpholino) propane sulphonate, biphenyl sulphonate, isethionate, 1-hydroxy-2-naphthalenesulfenesulfonate, dihydric phosphate, potassium hydrogen phosphate, dipotassium phosphate, potassium phosphate, sodium hydrogen phosphate, disodium phosphate, sodium phosphate, sodium dihydrogen phosphate, calcium phosphate, tertiary calcium phosphate, hexafluoro phosphate, ethenyl phosphate, 2-carboxylethyl phosphate or phenyl phosphate.

A synthetic method for a compound of formula XIII may be as follows: reacting 5-trifluoromethyl-2(1H)pyridone with nitro-substituted fluorobenzene, with DMSO as solvent, potassium carbonate as acid-binding agent and sodium iodide as catalyst so as to form nitro substitute; reducing the nitro substitute by iron powder in the presence of hydrochloric acid to prepare a simple amino-substituted compound; and preparing target products according to different compounds, shown in reaction formula I.

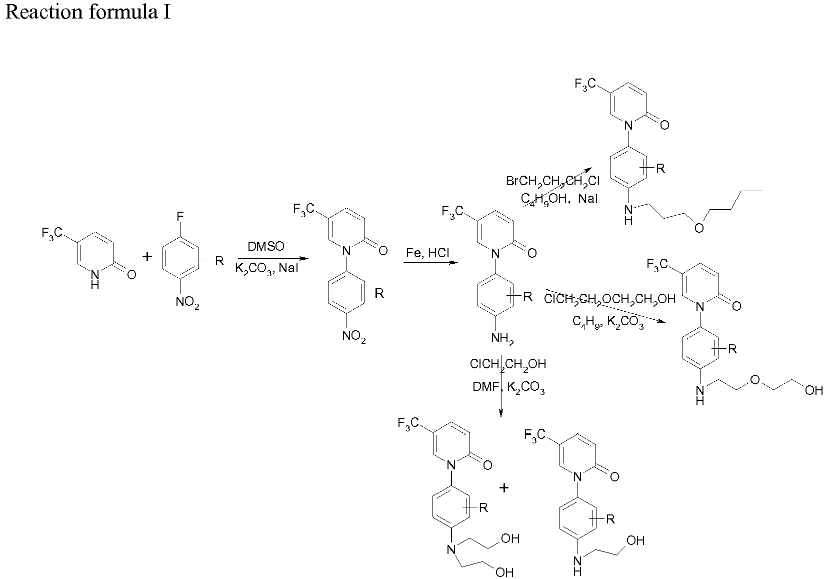

A compound, in which an amino group is bonded to a heterocyclic ring through an aliphatic side chain, is prepared by firstly reacting bromochloropropane with heterocyclic compound to produce chloroalkyl heterocyclic compound; and then reacting with the amino substitute prepared according to reaction formula I to obtain the target product, catalyzed by microwave, with normal butanol as solvent and sodium iodide as catalyst, shown in the reaction formula II. 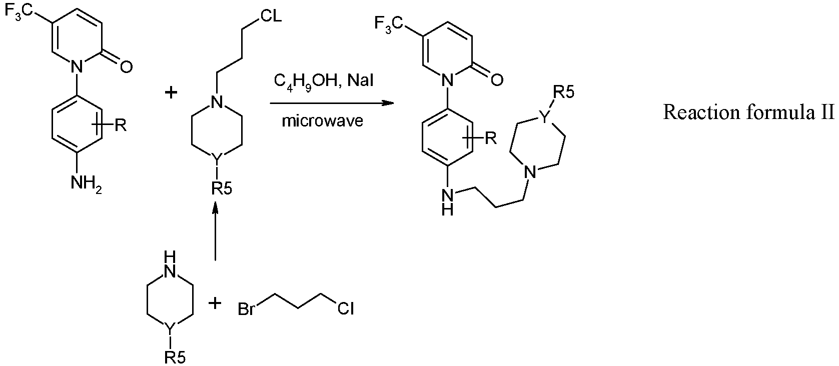 or, the target product is prepared by reacting hydroxyethyl amino substitute prepared a ccording to reaction formula I with thionyl chloride to produce chloroethyl amino subs titute; and then reacting with the heterocyclic compound, shown in reaction formula II I.

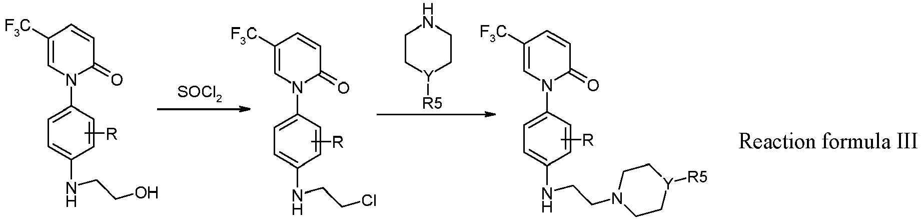

The synthetic starting product trifluoromethyl pyridone is a commercial material.

The above-mentioned compound is used for preparing a broad-spectrum medicament for fibrosis.

In the invention, based on the prior art, a substituted amino group is introduced onto the benzene ring at 1-position of pyridone; a hydrophilic group such as hydroxyl group and heterocyclic ring is introduced onto the amino group through an alkyl chain, thus obtaining a class of new pyridone compounds and salts thereof. The activity of the compounds is greatly enhanced.

The applicant finds that the produced compounds have relatively higher effects than the conventional pyridone compound by modifying the phenyl group by the substituted amino group on the basis of 1-phenyl-5-trifluoromethyl-pyridone; simultaneously the compounds including heterocyclic rings could be produced into various salts which are beneficial to being prepared into various liquid formulations.

The applicant figures out through experiment that the compounds provided by the invention have the anti-fibrosis pharmacological action as good as the pyridone compound in prior art, but have the significantly stronger effect than the pirfenidone in prior art over 60 times. Therefore, the invention also provides compounds represented by formula XIII as defined in the claims for use as an anti-fibrosis medicament.

### BRIEF DESCRIPTION OF DRAWINGS

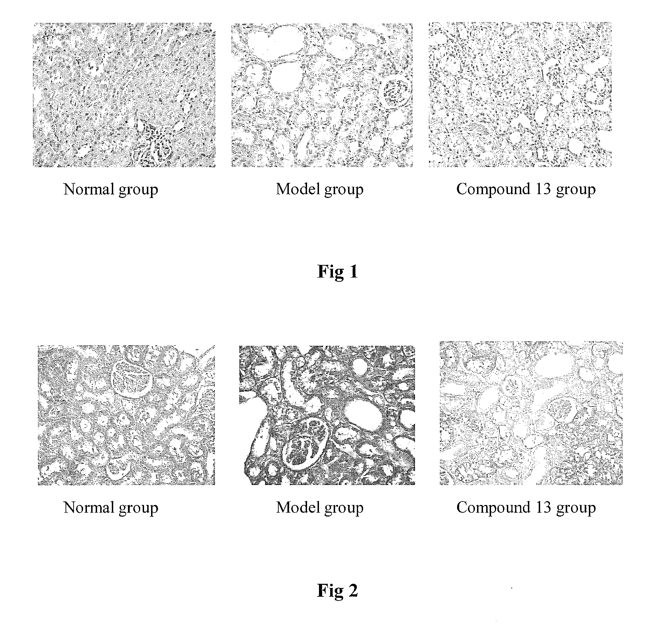

Figure 1 HE staining for renal pathology in embodiment 15 (×200)
Figure 2 Masson staining for renal pathology in embodiment 15 (×200)

### DETAILED DESCRIPTION OF PREFERRED EMBODIMENTS

### Example 1

### Preparation of 1-(2-chloro-4-((3-(4-methylpiperazin-1-yl)propyl)amino)phenyl)-5-(trifluoro methyl)pyridin-2(1H)-one

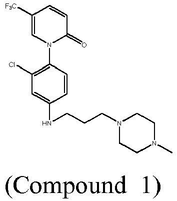

### A. Preparation of 1-(2-chloro-4-nitrophenyl)-5-(trifluoromethyl)pyridin-2(1H)-one

The preparation of 1-(2-chloro-4-nitrophenyl)-5-(trifluoromethyl)pyridin-2(1H)-one in cludes steps of: adding 8.2g (0.050mol) of 5-(trifluoromethyl)pyridin-2(1H)-one in 100 ml of DMSO for dissolving; adding 13.1g (0.075mol) of 3-chloro-4-fluoronitrobenzene, 11.0g (0.080mol) of potassium carbonate and 1.4g of sodium iodide and allowing the resulting system to react at 130 °C for 4 hours under stirring; after reaction, cooling to 40 °C; adding 100ml of 12% ammonia solution; separating out a great amount of precipitate; filtering; dissolving the filter residue with ethyl acetate; decolorizing by a ctive carbon; filtering; drying the filtrate by anhydrous sodium sulfate; filtering out so dium sulfate; reclaiming solvent; filtering to obtain the product of 1-(2-chloro-4-nitroph enyl)-5-(trifluoromethyl)pyridin-2(1H)-one. The product is brown solid of 12.0g; m.p.: 2 17.7∼218.3 °C. MS(m/z): 318(M⁺). ¹H-NMR(CDCl₃,300MHz)δppm: 6.769∼6.800(d,1H, Ar-H, J=3.3Hz), 7.579∼7.570(t,3H,Ar-H), 8.296∼8.333(dd,1H,J=3.3Hz,8.7Hz,Ar-H),8.492 (s,1H,Ar-H).

### B. Preparation of 1-(4-amino-2-chlorophenyl)-5-(trifluoromethyl)pyridin-2(1H)-one

The preparation of 1-(4-amino-2-chlorophenyl)-5-(trifluoromethyl)pyridin-2(1H)-one inclu des steps of: heating 12.0g (0.035mol) of 1-(2-chloro-4-nitrophenyl)-5-(trifluoromethyl)p yridin-2(1H)-one, 200mL of 50% ethanol and 5.8g (0.105mol) of reductive iron powde r to reflux; slowly adding 0.42mL (0.004mol) of concentrated HCI in dropwise way (d ropping after dilution by 5mL of 50% ethanol); refluxing for 5 hours under stirring; a fter reaction, regulating pH value to 10 by 15% KOH ethanol solution; filtering; wash ing the filter residues by 95% ethanol (2*10mL); extracting by ethyl acetate (50mL*3) after evaporating ethanol from the filtrate; drying the organic phase by anhydrous sod ium sulfate overnight; filtering; and evaporating filtrate to obtain the product of 1-(4-a mino-2-chlorophenyl)-5-(trifluoromethyl)pyridin-2(1H)-one. The product is khaki solid p owders of 10.2g. m.p.: 136∼138 °C. EI-MS(m/z): 288[M]⁺. ¹H-NMR(CDCl₃,300MHz)δ ppm: 3.559(br,2H,-NH2),6.633∼6.670(dd,1H,J=2.7 Hz,8.7HzAr-H),6.708∼6.740(d,1H, J=9. 6Hz,Ar-H),6.820∼6.828(d,1H,2.4Hz,Ar-H),7.089∼7.117(d,1H,J=2.4Hz,Ar-H),7.503∼7.544(dd, 1H,2.7Hz,9.6Hz,Ar-H),7.595(s,1H,Ar-H).

### C. Preparation of 1-(3-chloropropyl)-4-methylpiperazine

The preparation of 1-(3-chloropropyl)-4-methylpiperazine includes steps of: ice-bathing 0.1mol of piperidine, 100mL of acetone and 0.125mol of sodium hydrate (25%) below 5 °; slowly adding 0.1mol of 1-chloro-3-bromopropane in dropwise way; reacting for 48 hours at room temperature 25 °C; vacuum-evaporating solvent to dryness; adding 50mL of water; extracting by methylene dichloride (3*50mL); combining organic phases; drying by sodium sulphate over night; filtering; vacuum-evaporating to get oily product; adding concentrated hydrochloric acid in dropwise way to regulate pH value to 1-2; adding methylene dichloride and beating to remove 1-chloro-3-bromopropane; dissolving filter residue by adding an amount of water; regulating pH value to 12 by 25% sodium hydroxide; extracting by methylene dichloride (20mL*3); drying by sodium sulphate; filtering and vacuum-evaporating to obtain yellow oily product with a yield of 14.2%.¹H-NMR(CDCl₃,300MHz)δ1.930∼1.999(m,2H,-CH2-),2.301(s,3H,-CH3),2.470∼2.517 (m,10H,-CH2-),3.575∼3.619(t,2H,-CH2-).

### D. Preparation of 1-(2-chloro-4-((3-(4-methylpiperazin-1-yl)propyl)amino)phenyl)-5-(trifluoromethyl)pyridin-2 (1H)-one

The preparation of 1-(2-chloro-4-((3-(4-methylpiperazin-1-yl)propyl)amino)phenyl)-5-(trifluoromethyl)pyridin-2 (1H)-one includes steps of: adding 15mL of normal butanol to dissolve 2.59g (0.003mol) of 1-(4-amino-2-chlorophenyl)-5-(trifluoromethyl)pyridin-2(1H)-one; adding 0.528g(0.001mol) of 1-(3-chloro)propyl-4-methylpiperazine and uniformly mixing; and feeding a catalytic amount of potassium iodide; carrying out microwave reaction at 170 °C; filtering; removing solvent from the filtrate through evaporating; and separating residue by column chromatography with eluent of petroleum ether and ethyl acetate with proportion of 1:1 (1% triethylamine) to obtain yellow solid of 0.15g. m.p.: 129∼132 °C. ESI- MS(m/z): 429[M+H]⁺. 1H-NMR(CDCl₃,300MHz)δppm1.805∼1.845(m,2H,-CH2-),2.369(s,3H,-CH3),2.534∼2.575(t, 10H,-CH2-),3.201(br,2H,-CH2-),5.501(br,1H,-NH-),6.516∼6.553(dd,1H,*J*=2.4Hz,8.7Hz,Ar-H ),6.678∼6.734(dd,1H,*J*=2.4Hz,7.2Hz,Ar-H),7.071∼7.100 ( d,1H,*J*=8.7Hz,Ar-H), 7.491∼7.532(dd,1H,*J*=2.7Hz,9.6Hz,Ar-H), 7.604(s,1H,Ar-H).

### Example 2

### Preparation of 1-(2-chloro-4-((3-morpholinylpropyl)amino)phenyl)-5-(trifluoromethyl)pyridi n-2(1H)-one

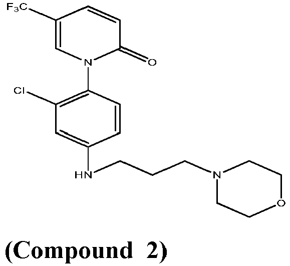

The preparation of 1-(2-chloro-4-((3-morpholinylpropyl)amino)phenyl)-5-(trifluoromethyl)pyridin-2(1H)-one includes steps of: adding 5mL of normal butanol to dissolve 0.54g (0.003mol) of 1-(4-amino-2-chlorophenyl)-5-(trifluoromethyl)pyridin-2(1H)-one; adding 0.528g(0.001mol) of 1-(3-chloro)propyl-morpholine and a catalytic amount of potassium iodide for uniformly mixing; carrying out microwave reaction at 180 °C; filtering, evaporating filtrate to dryness; and separating residues by chromatography with eluent of petroleum ether and ethyl acetate with proportion of 1:1 (1% triethylamine) to obtain yellow solid of 0.16g. m.p.: 95-97 °C. ESI-MS(m/z): 416[M+H]⁺. ¹H-NMR(CDCl₃,300MHz)δppm : 1.836∼1.856(m,2H,-CH2-), 2.527(br,6H,-CH2-),3.202∼3.258(t,2H,-CH2-),3.777(br,4H,-CH2-),5.403(br,1H,-NH-),6.523∼ 6.559(dd,1H,*J*=2.4Hz,8.7Hz,Ar-H),6.689∼6.698(d,1H,*J*=2.7Hz,Ar-H),6.737(s,1H,Ar-H),7.07 8∼7.138d,1H,*J*=8.7Hz,Ar-H) ,7.493∼7.534(dd,1H,*J*=2.7Hz,9.9Hz,Ar-H),7.604(s,1H,Ar-H).

### Example 3

### Preparation of 1-(2-chloro-4-((3-piperidin-1-yl)propylamino)phenyl)-5-(trifluoromethyl)pyri din-2(1H)-one

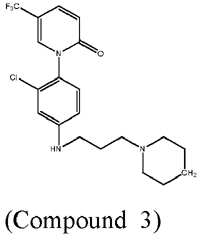

The preparation of 1-(2-chloro-4-((3-piperidin-1-yl)propylamino)phenyl)-5-(trifluoromethyl)pyridin-2(1H)-one incudes steps of: adding 15mL of normal butanol to dissolve 3.50g(0.012mol) of 1-(4-amino-2-chlorophenyl)-5-(trifluoromethyl)pyridin-2(1H)-one; adding 0.528g(0.004mol) of 1-(3-chloro)propylpiperidine for uniformly mixing; and adding a catalytic amount of potassium iodide; carrying out microwave reaction at 180 °C; filtering; evaporating filtrate to dryness; and separating residue by column chromatography with eluent of petroleum ether and ethyl acetate with proportion of 1:1 (1% triethylamine) to obtain light brown solid of 0.21g. m.p. : 112∼115 °C. EI-MS(m/z) : 413[M]⁺. ¹H-NMR(CDCl₃,300MHz)δppm : 1.482∼1.489(m,2H) ,1.607~1.642(m.4H),1.736~1.843(m,2H),2.425~2.491(m,6H),3.185(br,2 H),6.011(br,1H-NH-),6.499∼6.537(dd,1H,*J*=2.7Hz,8.7Hz,Ar-H),6.654-6.662(d,1H,*J*=2.4Hz, Ar-H),6.698~7.731(d,1H,*J*=9.9Hz,Ar-H),7.059~7.088(d,1H,*J*=8.7Hz,Ar-H),7.483~7.524(dd,1 H,*J*=2.7Hz,9.9Hz,Ar-H), 7.607(s,1H,Ar-H).

### Reference example 4

### Preparation of 1-(4-((3-butoxypropyl)amino)-2-chlorophenyl)-5-(trifluoromethyl)pyridin-2(1 H)-one

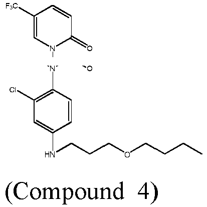

The preparation of 1-(4-((3-butoxypropyl)amino)-2-chlorophenyl)-5-(trifluoromethyl)pyridin-2(1H)-one incudes steps of: adding 15mL of normal butanol to dissolve 2.88g(0.01mol) of 1-(4-amino-2-chlorophenyl)-5-(trifluoromethyl)pyridin-2(1H)-one; adding 3.14g(0.02mol) of 1-chloro-3-bromopropane for uniformly mixing; and feeding a catalytic amount of potassium iodide; carrying out microwave reaction at 180 °C; filtering; evaporating filtrate to dryness; and separating residue by column chromatography with eluent of petroleum ether and ethyl acetate with proportion of 3:1 (1% triethylamine) to obtain white-alike solid of 0.20g. m.p.: 83.0∼85.0 °C. ESI-MS(m/z): 425[M+Na]⁺. ¹H-NMR(CDCl₃,300MHz)δppm: 0.921∼0.970 ( t, 3H, -CH3 ), 1.364∼1.439(m,2H,-CH2-),1.563∼1.612(m,2H,-CH2-),1.880∼1.919 (m,2H,-CH2-),3.213~3.255(t,H,-CH2-),3.415~3.458(t,2H,-CH2-),3.542~3.579(t,2H,-CH2-),4. 696(br,1H,-NH-),6.508~6.545(dd,1H,*J*=2.4Hz,2.4Hz,Ar-H),6.680~6.689(d,1H,*J*=2.7Hz,Ar-H ),.704~6.736(d,1H,*J*=9.6Hz,Ar-H),7.070~7.099(d,1H,*J*=8.7Hz,Ar-H),7.491~7.532(dd,1H,*J*=2

### Reference example 5

### Preparation of 1-(2-chloro-4-((2-hydroxyethyl)amino)phenyl)-5-(trifluoromethyl)pyridin-2(1H)-one

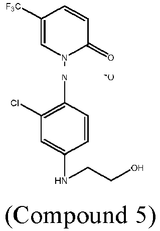

The preparation of 1-(2-chloro-4-((2-hydroxyethyl)amino)phenyl)-5-(trifluoromethyl)pyridin-2(1H)-one includes steps of: adding 12mL of chloroethanol and 12mL of DMF to dissolve 0.57g(0.002mol) of 1-(4-amino-2-chlorophenyl)-5-(trifluoromethyl)pyridin-2(1H)-one; adding 0.56g(0.004mol) of potassium carbonate; mixing for reaction for 12 hours at 130 °C; filtering; evaporating filtrate to dryness; and separating residue by column chromatography with eluent of petroleum ether and ethyl acetate with proportion of 1:1 to obtain brown solid of 0.080g. m.p. : 161.0~164.0 °C. EI-MS(m/z) : 332[M] ⁺. ¹H-NMR(CDCl₃,300MHz)δppm : 3.504∼3.543(t,2H,-CH2-),3.658∼3.709(t,2H-CH2-),4.412(br,1H,-NH-),6.590∼6.627(dd,1H,*J*= 2.7Hz,2.4Hz,Ar-H),710~6.742(d,1H,*J*=9.6Hz,Ar-H),6.754~6.762(d,1H,*J*=2.4Hz,Ar-H),7.128 ~7.157(d,1H,*J*=8.7Hz,Ar-H),7.500~7.542(dd,1H,*J*=2.7Hz,9.6Hz,Ar-H),7.597(s, 1H,Ar-H).

### Reference example 6

### Preparation of 1-(4-(N,N-(2-hydroxyethyl)amino)-2-chlorophenyl)-5-(trifluoromethyl)pyridin-2(1H)-one

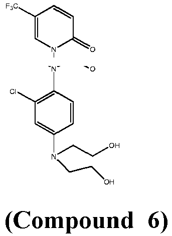

The preparation of 1-(4-(N,N-(2-hydroxyethyl)amino)-2-chlorophenyl)-5-(trifluoromethyl)pyridin-2(1H)-one includes steps of: adding 12mL of chloroethanol and 12mL of DMF to dissolve 0.57g(0.002mo1) of 1-(4-amino-2-chlorophenyl)-5-(trifluoromethyl)pyridin-2(1H)-one; adding 0.56g(0.004mol) of potassium carbonate; mixing for reaction for 12 hours at 130 °C; filtering; evaporating filtrate to dryness; and separating residue by column chromatography with eluent of petroleum ether and ethyl acetate with proportion of 1:1 to obtain red brown solid of 0.070g. m.p. : 169.0∼172.0 °C. EI-MS ( m/z ) : 376[M] ⁺. ¹H-NMR(CDCl₃,300MHz)δppm:3.213∼3.245(t,4H,-CH2-),3.661∼3,754(t,4H-CH2-),6.714∼6. 746(d,1H,*J*=9.6Hz,Ar-H),6.864∼6.903(dd,1H,*J*=2.7Hz,9.6Hz,Ar-H),7.018∼7.027(d,1H,*J*=2.7 Hz,Ar-H),7.214∼7.244(d,1H,*J*=9.0Hz,Ar-H),7.505∼7.514(dd,1H,*J*=2.7Hz,Ar-H).

### Example 7

### Preparation of 1-(2-chloro-4-(((3-piperidin-1-yl)propyl)amino)phenyl)-5-(trifluoromethyl)p yridin-2(1H)-one hydrochloride

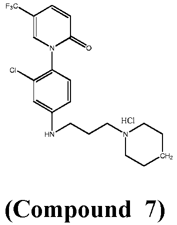

The preparation of 1-(2-chloro-4-(((3-piperidin-1-yl)propyl)amino)phenyl)-5-(trifluoromethyl)pyridin-2(1H)-one hydrochloride includes steps of: dissolving 2.9mmol of 1-(4-(((3-piperidin-1-yl)propyl)amino)-2-chlorophenyl)-5-(trifluoromethyl)pyridin-2(1H)-one by an amount of ethanol; adding 2mmol of hydrochloric acid; mixing for reaction for 2 hours; evaporating solvent to dryness to obtain 1-(2-chloro-4-(((3-piperidin-1-yl)propyl)amino)phenyl)-5-(trifluoromethyl)pyridin-2(1H)-one hydrochloride which is white-alike solid of 0.12g. M.P.: 192∼195 °C. EI-MS(m/z) : 414[M+H]⁺. ¹H-NMR(D₂O)δppm : 1.343∼1.718(m,6H,-CH2-),1.857∼1.905(2H,-H), 1.956∼2.055(m,2H,-CH2-),2.829∼2.905(t,2H ,-CH2-),3.122∼3.116(t,2H,-CH2-),3.221-3.284(2H-CH2-),3.445∼3.487(2H-CH2-),6.764∼6.8 12(2H,Ar-H),6.965∼6.972(1H,Ar-H),7.199∼7.228(1H,Ar-H),7.785∼7.907(1H,Ar-H),8.075(1 H,Ar-H).

### Reference example 8

### Preparation of 1-(2-chloro-4-((2-(2-hydroxyethoxy)ethyl)amino)-phenyl)-5-(trifluoromethyl) pyridin-2(1H)-one

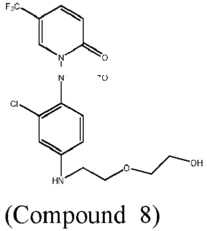

The preparation of 1-(2-chloro-4-((2-(2-hydroxyethoxy)ethyl)amino)-phenyl)-5-(trifluoromethyl)pyridin-2(1H)-o ne includes steps of: dissolving 1-(4-amino-2-chloro)phenyl-5-(trifluoromethyl)pyridin-2(1H)-one and 28mmol of chloroethoxy ethanol in 50mL of normal butanol; adding 1.9mmol of potassium carbonate; carrying out refluxing reaction for 72 hours; filtering; evaporating filtrate to dryness; and separating by column chromatography with eluent of petroleum ether and ethyl acetate with proportion of 1:1 to obtain yellow oily product of 0.33g. EI-MS(m/z) : 376[M]⁺, ¹H-NMR(CDCl₃,300MHz)δppm : 3.320∼3.355(t,2H,-CH2-),3.607∼3,637(t,2H,-CH2-),3.714∼6.748((t,2H,-CH2-),3.768∼3.798(( t,2H,-CH2-),6.609∼6.646(dd,1H,J=2.4Hz,8.4Hz,Ar-H),6.710-6.742(d,1H,J=9.6Hz,Ar-H),6.77 5-6.783(d,1H,J=2.4Hz,Ar-H),7.107∼7.136(d,1H,J=8.7Hz,Ar-H),7.501∼7.542(dd,1H,J=2.7Hz, 9.6Hz,Ar-H),7.603(s, 1H,Ar-H).

### Example 9

### Preparation of 1-((4-((piperazin-1-yl)ethyl)amino)-2-chlorophenyl)-5-(trifluoromethyl)pyridi n-2(1H)-one

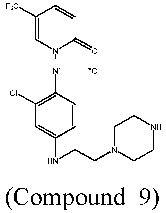

### A. Preparation of 1-(2-chloro-4-((2-chloroethyl)amino)-phenyl)-5-(trifluoromethyl)pyridin-2(1H)-one

The preparation of 1-(2-chloro-4-((2-chloroethyl)amino)-phenyl)-5-(trifluoromethyl)pyridin-2(1H)-one includes steps of: mixing 3mmol of 1-(2-chloro-4-((2-hydroxyethyl)amino)phenyl)-5-(trifluoromethyl)pyridin-2(1H)-one, 120mL of methylene dichloride, 4.5mmol of thionyl chloride and 4.5mmol of triethylamine for reaction for 28 hours at room temperature; and separating by column chromatography with eluent of petroleum ether and ethyl acetate with proportion of 3:1 to obtain straw yellow solid of 0.5g. M.P.: 160.0∼162.0 °C. EI-MS (m/z): 350[M]⁺. ¹H-NMR(CDCl₃,300MHz) δppm: 3.502∼3.541(t,2H,-CH2-),3.713∼3.752(t,2H,-CH2-),6.909∼6.647(dd,1H,J=2.7Hz,8.7Hz,Ar-H ),6.716~6.777(2H,Ar-H),7.135~7.164(d,1H,J=8.7Hz,Ar-H),7.508~7.550(dd,1H,J=2.7Hz,9.6H z,Ar-H),7.600(s, 1H,Ar-H).

### B.

Preparation of 1-(2-chloro-4-((2-piperazin-1-yl)ethyl)amino)-phenyl)-5-(trifluoromethyl)pyridin-2(1H)-one dissolving 1.3mmol of 1-(2-chloro-4-((2-chloroethyl)amino)phenyl)-5-(trifluoromethyl)pyridin-2(1H)-one and 7.8mmol of anhydrous piperazine in 50mL of acetonitrile; adding an amount of sodium iodide; carrying out refluxing reaction for 12 hours; filtering; evaporating filtrate to dryness; and separating by column chromatography with eluent of ethyl acetate and methanol with proportion of 5:1 1 (2% triethylamine) to obtain yellow colloid substance of 0.32g. EI-MS(m/z): 400[M] ⁺, ¹H-NMR(CDCl₃,300MHz)δppm: 2.442(s,4H,-CH2-),2.628(s,2H, -CH2-),2.904(s,4H,-CH2-),3.144~3.158(d,2H,-CH2-),4.776(s,1H-NH-),6.572~6.60(d,1H,J=8. 4Hz,Ar-H),6.707~6.736(d,1H,J=8.7Hz,Ar-H),7.094~7.122(d,1H,J=8.4Hz,Ar-H),7.500~7.530( d,1H,J=9.0Hz,Ar-H),7.609(s,1H,Ar-H).

### Example 10

### Preparation of 1-(2-chloro-4-((2-(piperidyl-1-yl)ethyl)amino)phenyl)-5-(trifluoromethyl)pyri din-2(1H)-one

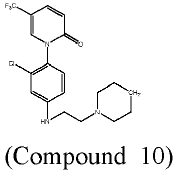

The preparation of 1-(2-chloro-4-((2-(piperidyl-1-yl)ethyl)amino)phenyl)-5-(trifluoromethyl) pyridin-2(1H)-one includes steps of: dissolving 1.7mmol of 1-(2-chloro-4-((2-chloroethyl) amino)phenyl)-5-(trifluoromethyl)pyridin-2(1H)-one and 10.3mmol of piperazine in 50m L of acetonitrile; adding an amount of sodium iodide; carrying out refluxing reaction f or 17 hours; filtering; evaporating filtrate to dryness; and separating by column chrom atography with eluent of petroleum ether and ethyl acetate with proportion of 1:1 to o btain yellow colloid substance of 0.34g. EI-MS(m/z): 399[M] ⁺, 1H-NMR(CDCl₃,300M Hz)δppm: 1.470∼1.487(d,2H,J=5.1,-CH2-),1.576∼1.647(m,4H,-CH2-),2.436(s,4H,-CH2-),2. 604∼2.644(t,2H,-CH2-),3.152∼3.165(d,2H,-CH2-),4.941(s,1H,-NH-),6.568∼6.605(dd,1H,J=2. 4Hz,8.7Hz,Ar-H),6.708∼6.734(t,1H,Ar-H),7.088∼7.117(d,1H, J=8.7Hz,Ar-H),7.493∼7.502(d, 1H,J=2.7Hz,Ar-H),7.525∼7.534(d,-H,J=2.7Hz,Ar-H).

### Example 11

### Preparation of 1-(2-chloro-4-((2-morpholinylethyl)amino)phenyl)-5-(trifluoromethyl)pyridin -2(1H)-one

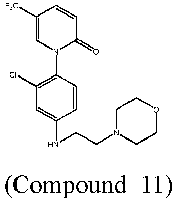

The preparation of 1-(2-chloro-4-((2-morpholinylethyl)amino)phenyl)-5-(trifluoromethyl)pyridin-2(1H)-one includes stepsof: dissolving 1.7mmol of 1-(2-chloro-4-((2-chloroethyl)amino)phenyl)-5-(trifluoromethyl)pyridin-2(1H)-one and 10.9mmol of morpholine in 50mL of acetonitrile; adding an amount of sodium iodide; carrying out refluxing reaction for 24 hours; filtering; evaporating filtrate to dryness; and separating by column chromatography with eluent of petroleum ether and ethyl acetate with proportion of 1:1 to obtain yellow colloid substance of 0.67g. EI-MS(m/z): 401[M]⁺, ¹H-NMR(CDCl₃,300MHz)δppm2.500(s,4H,-CH2-),2.650∼2.688(t,2H,-CH2-),3.150∼3.204(m ,2H,-CH2-),3.728∼3.758(t,4H,-CH2-),4.781(s,1H),6.573∼6.610(dd,1H,J=2.4Hz,6.0Hz,Ar-H), 6.703∼6.743(t,2H,Ar-H),7.098∼7.127(d,1H,J=8.7Hz,Ar-H),7.494∼7.535(dd,1H,J=2.7Hz,9.6H z,Ar-H),7.603(s,1H,Ar-H).

### Example 12

### Preparation of 1-(2-chloro-4-((2-(4-methylpiperazin-1-yl)ethyl)amino)-phenyl)-5-(trifluorom ethyl)pyridin-2(1H)-one

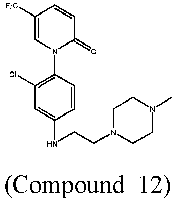

The preparation of 1-(2-chloro-4-((2-(4-methylpiperazin-1-yl)ethyl)amino)-phenyl)-5-(trifluoromethyl)pyridin-2( 1H)-one includes steps of: dissolving 1.7mmol of 1-(2-chloro-4-((2-chloroethyl)amino)phenyl)-5-(trifluoromethyl)pyridin-2(1H)-one and 10.9mmol of N-methyl piperazine in 50mL of acetonitrile; adding an amount of sodium iodide; carrying out refluxing reaction for 22 hours; filtering; evaporating filtrate to dryness; and separating by column chromatography with eluent of petroleum ether and ethyl acetate with proportion of 1:1 to obtain yellow solid of 0.70g. m.p.: 113.1∼115.2,EI-MS(m/z): 414[M] ⁺ , ¹H-NMR(CDCl₃,300MHz)δppm : 2.321(s,3H,-CH3),2.511(br,8H,-CH2-),2.639∼2.678(t,2H,-CH2-),3.126∼3.181(q,2H,-CH2-),4. 736∼4.765(t,1H,-NH-),6.566∼6.603(dd,1H,J=2.4Hz,8.7Hz,Ar-H),6.708∼6.740(t,2H,Ar-H),7.0 96∼7.125(d,1H, J=9.6Hz,Ar-H),7.496∼7.537(dd,1H,J=2.7Hz,9.6Hz,Ar-H),7.609(s,1H,Ar-H).

### Example 13

### Preparation of 1-(2-chloro-4-((2-(4-(2-hydroxyethyl)piperazin-1-yl)ethyl)amino)phenyl)-5-(trifluoromethyl) pyridin-2(1H)-one

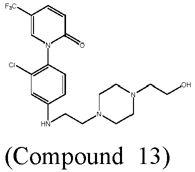

The preparation of 1-(2-chloro-4-((2-(4-(2-hydroxyethyl)piperazin-1-yl)ethyl)amino)phenyl)-5-(trifluoromethyl) pyridin-2(1H)-one includes steps of: dissolving 1.7mmol of 1-(2-chloro-4-((2-chloroethyl)amino)phenyl)-5-(trifluoromethyl)pyridin-2(1H)-one and 10.9mmol of hydroxyethyl piperazine in 50mL of acetonitrile; adding an amount of sodium iodide; carrying out refluxing reaction for 24 hours; filtering; evaporating filtrate to dryness; and separating by column chromatography with eluent of petroleum ether and ethyl acetate with proportion of 1:1 to obtain yellow colloid substance of 0.51g. EI-MS(m/z): 444[M]⁺. ¹H-NMR(CDCl₃,300MHz)δppm : 2.567∼2.691(m,12H, -CH2-),3.139∼3,192(t,2H,-CH2-),3.632∼3.667(t,2H,-CH2-),4.737(br,1H,-NH-),6.563∼6.600( dd,1H,J=2.4Hz,8.7Hz,Ar-H),6.702∼6.738(t,2H,Ar-H),7.094∼7.123(d,1H,J=8.7Hz,Ar-H),7.49 2∼7.533(dd,1H, J=2.7Hz,9.0Hz,Ar-H),7.603(s,1H,Ar-H).

### Example 14

### Inhibition test of compounds to NIH3T3 mechanocytes

An MTT method is used and comprises steps of: culturing cells in DMEM culture medium including 5% calf serum and preparing the cells into cell suspension of 3*10⁴/ml; inoculating in 96-pore plate according to 100 µl/pore; transferring new culture medium including compounds with different concentration, fluorofenidone and 1% calf serum after cells are adhered, wherein three repeated pores are provided for each concentration; respectively adding 100 µl of MTT solution in each pore after 48 hours and 72 hours of administrating (the culture medium is prepared into 5mg/ml and kept in dark after filtering), sucking out MTT after 4 hours; adding 150 µl of DMSO which is the dissolving liquid of MTT; after 10min and MTT is completely dissolved, measuring OD value by ELISA reader; calculating IC₅₀ values of fluorofenidone and measured compounds according to inhibition ratio; figuring out multiple of activities of measured compounds and fluorofenidone according to IC₅₀ values of fluorofenidone and measured compounds; and obtaining relative IC₅₀ value of measured compounds according to multiple and IC₅₀ value of fluorofenidone on a certain plate.

### Inhibition activity of measured compounds to NIH3T3 mechanocyte

| Measured compounds | 48 hours | | 72 hours | |
|---|---|---|---|---|
| | Relative IC₅₀ (mM) | Multiple | Relative IC₅₀ (mM) | Multiple |
| | | | | |
| Fluorofenidone | 4.43 | | 3.52 | |
| Compound 1 | 0.286 | **15.50** | 0.163 | **21.60** |
| Compound 2 | 0.241 | **18.36** | 0.161 | **21.87** |
| Compound 3 | 0.238 | **18.60** | 0.065 | **54.0** |
| Compound 4 | 0.702 | **6.31** | 0.311 | **11.31** |
| Compound 5 | 1.380 | **3.21** | 0.632 | **5.57** |
| Compound 6 | 0.641 | **6.91** | 0.587 | **6.00** |
| Compound 7 | 0.259 | **17.09** | 0.049 | **71.17** |
| Compound 8 | 0.487 | **9.09** | 0.332 | **10.59** |
| Compound 10 | 0.214 | **20.73** | 0.062 | **56.50** |
| Compound 11 | 0.174 | **25.50** | 0.056 | **62.50** |
| Compound 12 | 0.330 | **13.42** | 0.106 | **33.33** |
| Compound 13 | 0.100 | **44.14** | 0.062 | **57.20** |

| | | | | |
|---|---|---|---|---|
| Notes: multiple is IC₅₀ value of compounds to IC₅₀ value of fluorofenidone | | | | |

### Example 15

Observation of treatment effect of compound 13 to rat unilateral ureteral obstruction renal fibrosis model

### Materials and methods

### 1. Experimental chemicals

The compound 13 is prepared according to the method provided by the invention.

### 2. Experimental animals

Nine male SD rats of 188-213g, coming from Hunan Slac Laobratory Animals Co., Ltd., are illuminated for 12 hours every day; feed is provided by Shanghai Slac Laobratory Animals Co., Ltd.; and drinking water is provided by Department of Laboratory Animal Science of Central South University.

### 3. Experimental methods

(1) **Randomization:** nine rats are divided into three groups at random, namely a normal group (n=3); a model group (n=3) and a treatment group (n=3) treated by compound 13 of 15mg/kg; three rats are in a hutch; and the experimental animals are adaptively fed for two days.

### (2) Unilateral ureteral obstruction modeling:

The unilateral ureteral obstruction modeling comprises steps of: lumbar-injecting each rat with 10% chloral hydrate according to 0.35ml/100g for anesthesia, fixing on a rat fixing plate; wetting the back skin by water, tightening the skin; unhairing by elbowed surgical scissors in a way closely attaching the skin; sterilizing drape in a conventional way; making an incision of 1.0cm in longitudinal direction at a junction of a position 1.0cm below left costal margin and 0.8cm next to median line of vertebral column; separating successive layers to expose left kidney and left ureter; tying off left ureter against lower pole of left kidney by a thread of 4.0 and another portion 1.0cm therebelow; isolating ureter between those two points; flushing abdominal cavity by gentamicin physiological saline solution; and stitching successive layers of retroperitoneal space and back skins after no leakage and hemorrhage.

**(3) Pharmacological intervention:** intragastric administration is carried out the day before modeling operation according to one time per day for 12 days; the method is detailed as follows:
a) preparing 0.5% CMCNa solution by adding an amount of 0.9% physiological saline into CMCNa powder and preparing following groups of chemicals with 0.5% CMCNa solution as solvent.
b) lavaging the normal group by 0.5% CMC-NA of 6ml/kg.d for one time per day.
c) lavaging the model group by 0.5% CMC-NA of 6ml/kg.d for one time per day.
d) lavaging the treatment group treated by compound 13 of 15mg/kg by 0.5% CMC-NA of
   6ml/kg.d for one time per day.

### (4) Animal sacrifice and sample collection

Each group of rats is respectively lumbar-injected with 10% chloral hydrate (0.7-0.9ml/100g) on 11st day after operation for excessive anesthesia until sacrifice, renal tissues on the obstruction side is fixed by 4% formaldehyde, embedded by paraffin and prepared into 4 mum-thick slices for HE staining and Masson staining.

### (5) HE staining evaluation standard:

HE staining slices of renal tissues are successively observed in fives fields of view of renal tubulointerstitium on upper left side, upper right side, lower left side, lower right side and middle portion by a low power lens and are evaluated according to eight indexes of renal interstitium lesion: renal tubular epithelial cell vacuolar degeneration, renal tubular ectasia, renal tubular atrophy, red cell cast, protein cast, interstitial edema, interstitial fibrosis and interstitial inflammatory cell infiltration; an average value is calculated as the index of renal tubulointerstitial lesion of the sample; and the evaluation standard is based on the reference of Radford MG Jr, Donadio JV Jr, Bergstralh EJ, et al. Predicting renal outcome in IgA nephropathy. J Am Soc Nephrol, 1997, 8(2):199-207.

### (6) Masson staining evaluation standard

Masson staining slices of renal tissues are observed in 20 fields of vision for each sample at random under 400X light microscope; percent of blue-stain collagens in the fields of vision is calculated; an average value is determined after semi-quantitative evaluation: no positive staining, 0; <25%, 1; 25-50%, 2; 50-75%, 3; >75 %, 4; and the evaluation standard is based on references. Lin SL, Chen RH, Chen YM, et al. Pentoxifylline Attenuates Tubulointerstitial Fibrosis by Blocking Smad3/4-Activated Transcription and Profibrogenic Effects of Connective Tissue Growth Factor. J Am Soc Nephrol.2005, 16: 2702-2713.

**4. Statistical methods**: analytical method of variance of single factor is adopted.

### Experimental Results

### 1. Pathological evaluation results of renal interstitium lesions through HE staining

**Table 1 comparison of indexes of renal tubulointerstitial lesions of obstruction kidneys of rats in groups**

| Group | Number | Score(**X̅**±**S**) |
|---|---|---|
| Normal group | 3 | 0.33±0.12 |
| Model group | 3 | 9.00±1.00^{★★★} |
| Compound 13 group | 3 | 7.00±0.35^{★★}** |

| | | |
|---|---|---|
| **Notes:** **comparison to normal group,** ^{★}pH0.05, ^{★★}p<0.01; ^{★★★}p<0.001; **comparison to model group,** *pH0.05, **p<0.01, ***p<0.001; | | |

### 2. Pathological evaluation results of renal interstitium lesions through MASSON staining

**Table 2 evaluation results of renal interstitium collagens of left kidneys of rats in groups through MASSON staining**

| Group | Number | Score(**X̅**±**S**) |
|---|---|---|
| Normal group | 3 | 0.25±0.00 |
| Model group | 3 | 2.45±0.38^{★★★} |
| Compound 13 group | 3 | 1.52±0.16^{★★}** |

| | | |
|---|---|---|
| **Notes:** **comparison to normal group,** *pH0.05, **p<0.01; ***p<0.001; **comparison to model group,** *pH0.05, **p<0.01, ***p<0.001; | | |

### Conclusion:

The compound 13 of 15mg/kg can effectively treat renal fibrosis.

## Claims

1. 1-(substituted phenyl)-5-trifluoromethyl-2(1H)pyridone compounds, having a formula (XIII), 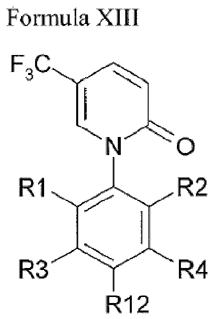
wherein, R1-R4, and R12 are selected from: H, CN, NO₂, hydroxyl group, amino group, halogen atom, C₁-C₆ alkoxyl group, NR¹⁰R¹¹, C(O)R^{14,} C₁-C₆ alkyl group,
C₁-C₆ haloalkyl group, C₂-C₆ alkenyl group, carboxyl group and carboxylic ether; wherein R¹⁴ is C₁-C₆ alkyl group, R¹⁰ and R¹¹ are selected from H, C₁-C₆ hydroxyalkyl group, esterified C₁-C₆ hydroxyalkyl group, C₁-C₆ alkoxyalkyl group, or Formula XIV;
and at least one of R1-R4,and R12 is NR¹⁰R¹¹ ; and at least one of R¹⁰ and R¹¹ is of the Formula XIV: 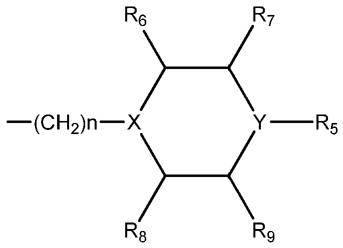
wherein R5 is selected from H, C₁-C₆ alkyl group, C₁-C₆ hydroxyalkyl group, esterified C₁-C₆ hydroxyalkyl group and C₂-C₆ alkenyl group; R6-R9 are selected from H, C₁-C₆ alkoxyl group, =O, C₁-C₄ alkyl group, C₁-C₄ haloalkyl group, C₁-C₄ hydroxyalkyl group, and C₂-C₄ alkenyl group; X is selected from N, CH₂; Y is selected from N, O, C; with the proviso that when Y is O, R⁵ is absent; n is 1-6;
and pharmaceutically acceptable salts thereof.

2. 1-(substituted phenyl)-5-trifluoromethyl-2(1H)pyridone compounds according to claim 1, wherein R12 is NR¹⁰R¹¹.

3. 1-(substituted phenyl)-5-trifluoromethyl-2(1H)pyridone compounds according to claim 1, wherein one of R1-R4 is a halogen atom and others are H if R12 is NR¹⁰R¹¹.

4. 1-(substituted phenyl)-5-trifluoromethyl-2(1H)pyridone compounds according to claim 1, wherein the compounds are:
1-((2-chloro-4-((3-(4-methylpiperazin-1-yl)propyl)amino)phenyl)-5-(trifluoromethyl)pyridin-2 (1H)-one (compound 1);
1-((2-chloro-4-((3-morpholinylpropyl)amino)phenyl)-5-((trifluoromethyl)pyridin-2(1H)-one (compound 2);
1-((2-chloro-4-((3-piperidin-1-yl)propylamino)phenyl)-5-(trifluoromethyl)pyridin-2(1H)-one (compound 3);
1-((2-chloro-4-(((3-piperidin-1-yl)propyl)amino)phenyl)-5-(trifluoromethyl)pyridin-2(1H)-one hydrochloride (compound 7);
1-(((4-((piperazin-1-yl)ethyl)amino)-2-chlorophenyl)-5-((trifluoromethyl)pyridin-2(1H)-one (compound 9);
1-((2-chloro-4-((2-(piperidyl-1-yl)ethyl)amino)phenyl)-5-(trifluoromethyl)pyridin-2(1H)-one (compound 10);
1-((2-chloro-4-((2-morpholinylethyl)amino)phenyl)-5-((trifluoromethyl)pyridin-2(1H)-one (compound 11);
1-((2-chloro-4-((2-(4-methylpiperazin-1-yl)ethyl)amino)phenyl)-5-(trifluoromethyl)pyridin-2( 1H)-one (compound 12);
1-((2-chloro-4-((2-(4-(2-hydroxyethyl)piperazin-1-yl)ethyl)amino)phenyl)-5-((trifluoromethyl) pyridin-2(1H)-one (compound 13).

5. A compound of any one of claims 1 to 4 for use as an anti-fibrosis medicament.

## Patentansprüche

1. 1-(Substituierte Phenyl)-5-trifluormethyl-2(1H)pyridonverbindungen mit einer Formel (XIII), 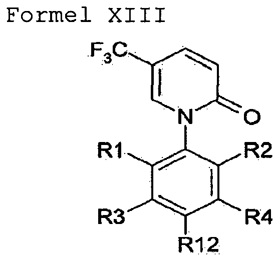
worin R1-R4 und R12 ausgewählt sind aus: H, CN, NO₂, Hydroxylgruppe, Aminogruppe, Halogenatom, C₁-C₆-Alkoxylgruppe, NR¹⁰R¹¹, C(O)R¹⁴, C₁-C₆-Alkylgruppe, C₁-C₆-Halogenalkylgruppe, C₂-C₆-Alkenylgruppe, Carboxylgruppe und Carbonsäureether; worin R¹⁹ eine C₁-C₆-Alkylgruppe darstellt, R¹⁰ und R¹¹ ausgewählt sind aus H, C₁-C₆-Hydroxyalkylgruppe, veresterter C₁-C₆-Hydroxyalkylgruppe, C₁-C₆-Alkoxyalkylgruppe oder Formel XIV;
und mindestens eines von R1-R4 und R12 für NR¹⁰R¹¹ steht; und mindestens eines von R¹⁰ und R¹¹ die Formel XIV : 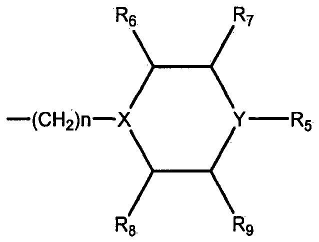
hat, worin R5 ausgewählt ist aus H, C₁-C₆-Alkylgruppe, C₁-C₆-Hydroxyalkylgruppe, veresterter C₁-C₆-Hydroxyalkylgruppe und C₂-C₆Alkenylgruppe; R6-R9 ausgewählt sind aus H, C₁-C₆-Alkoxylgruppe, =O, C₁-C₄-Alkylgruppe, C₁-C₄-Halogenalkylgruppe, C₁-C₄-Hydroxyalkylgruppe und C₂-C₄-Alkenylgruppe; X ausgewählt ist aus N, CH₂; Y ausgewählt ist aus N, O, C; mit der Maßgabe, dass wenn Y für O steht, R⁵ abwesend ist; n 1-6 ist;
und pharmazeutisch annehmbare Salze davon.

2. 1-(Substituierte Phenyl)-5-trifluormethyl-2(1H)pyridonverbindungen gemäß Anspruch 1, worin R12 für NR¹⁰R¹¹ steht.

3. 1-(Substituierte Phenyl)-5-trifluormethyl-2(1H)pyridonverbindungen gemäß Anspruch 1, worin eines von R1-R4 ein Halogenatom darstellt und andere für H stehen, falls R12 für NR¹⁰R¹¹ steht.

4. 1-(Substituierte Phenyl)-5-trifluormethyl-2(1H)pyridonverbindungen gemäß Anspruch 1, wobei die Verbindungen sind:
1-((2-Chlor-4-((3-(4-methylpiperazin-1-yl)propyl)amino)phenyl)-5-(trifluormethyl)pyridin-2-(1H)-on (Verbindung 1);
1-((2-Chlor-4-((3-morpholinylpropyl)amino)phenyl)-5((trifluormethyl)pyridin-2(1H)-on (Verbindung 2);
1-((2-Chlor-4-((3-piperidin-1-yl)propylamino)phenyl)-5trifluormethyl)pyridin-2(1H)-on (Verbindung 3);
1-((2-Chlor-4-(((3-piperidin-1-yl)propyl)amino)phenyl)-5(trifluormethyl)pyridin-2(1H)-on-hydrochlorid (Verbindung 7);
1-(((4-((Piperazin-1-yl)ethyl)amino)-2-chlorphenyl)-5((trifluormethyl)pyridin-2(1H)-on (Verbindung 9);
1-((2-Chlor-4-((2-(piperidyl-1-yl)ethyl)amino)phenyl)-5(trifluormethyl)pyridin-2(1H)-on (Verbindung 10);
1-((2-Chlor-4-((2-morpholinylethyl)amino)phenyl)-5((trifluormethyl)pyridin-2(1H)-on (Verbindung 11);
1-((2-Chlor-4-((2-(4-methylpiperazin-1yl)ethyl)amino)phenyl)-5-(trifluormethyl)pyridin-2(1H)-on (Verbindung 12);
1-((2-Chlor-4-((2-(4-(2-hydroxyethyl)piperazin-1yl)ethyl)amino)phenyl)-5-((trifluormethyl)pyridin-2(1H)on (Verbindung 13).

5. Verbindung nach einem der Ansprüche 1 bis 4 zur Verwendung als Anti-Fibrose-Medikament.

## Revendications

1. Composés de 1-(phényl substitué)-5trifluorométhyl-2(1H)pyridone, possédant la formule (XIII), 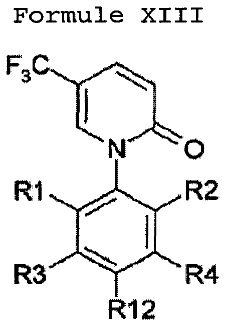
dans laquelle R1 à R4 et R12 sont choisis parmi : H, CN, NO₂, un groupe hydroxyle, un groupe amino, un atome d'halogène, un groupe alcoxyle en C₁ à C₆, NR¹⁰R¹¹, C(O)R¹⁴, un groupe alkyle en C₁ à C₆, un groupe haloalkyle en C₁ à C₆, un groupe alcényle en C₂ à C₆, un groupe carboxyle et un éther carboxylique ; où R14 est un groupe alkyle en C₁ à C₆, R¹⁰ et R¹¹ sont choisis parmi H, un groupe hydroxyalkyle en C₁ à C₆, un groupe hydroxyalkyle en C₁ à C₆ estérifié, un groupe alcoxyalkyle en C₁ à C₆ ou la formule XIV ;
et au moins un parmi R1 à R4 et R12 est NR¹⁰R¹¹ ; et au moins un parmi R¹⁰ et R¹¹ est de formule XIV, 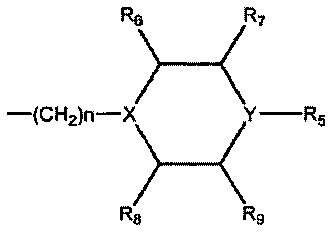
dans laquelle R5 est choisi parmi H, un groupe alkyle en C₁ à C₆, un groupe hydroxyalkyle en C₁ à C₆, un groupe hydroxyalkyle en C₁ à C₆ estérifié et un groupe alcényle en C₂ à C₆ ; R6 à R9 sont choisis parmi H, un groupe alcoxyle en C₁ à C₆, =O, un groupe alkyle en C₁ à C₄ , un groupe haloalkyle en C₁ à C₄, un groupe hydroxyalkyle en C₁ à C₄ et un groupe alcényle en C₁ à C₄ ; X est choisi parmi N, CH₂ ; Y est choisi parmi N, O, C ; à condition que quand Y est O, R⁵ soit absent ; n est 1 à 6 ;
et leurs sels pharmaceutiquement acceptables.

2. Composés de 1-(phényl substitué)-5-trifluorométhyl-2(1H)pyridone selon la revendication 1, dans lesquels R12 est NR¹⁰R¹¹.

3. Composés de 1-(phényl substitué)-5trifluorométhyl-2(1H)pyridone selon la revendication 1, dans lesquels un parmi R1 à R4 est un atome d'halogène et les autres sont H si R12 est NR¹⁰R¹¹.

4. Composés de 1-(phényl substitué)-5-trifluorométhyl-2(1H)pyridone selon la revendication 1, dans lesquels les composés sont :
la 1-(2-chloro-4-((3-(4-méthylpipérazin-1-yl)-propyl)amino)phényl)-5-(trifluorométhyl)pyridin-2(1H)-one (composé 1) ;
la 1-(2-chloro-4-((3-morpholinylpropyl)amino)-phényl)-5-(trifluorométhyl)pyridin-2(1H)-one (composé 2) ;
la 1-(2-chloro-4-((3-pipéridin-1-yl)propyl-amino)phényl)-5-(trifluorométhyl)pyridin-2(1H)-one (composé 3) ;
le chlorhydrate de 1-(2-chloro-4-(((3-pipéridin-1-yl)propyl)amino)phényl)-5-(trifluorométhyl)-pyridin-2(1H)-one (composé 7) ;
la 1-((4-((pipérazin-1-yl)éthyl)amino)-2-chlorophényl)-5-(trifluorométhyl)pyridin-2(1H)-one (composé 9) ;
la 1-(2-chloro-4-((2-(pipéridyl-1-yl)éthyl)-amino)phényl)-5-(trifluorométhyl)pyridin-2(1H)-one (composé 10) ;
la 1-(2-chloro-4-((2-morpholinyléthyl)amino)-phényl)-5-(trifluorométhyl)pyridin-2(1H)-one (composé 11) ;
la 1-(2-chloro-4-((2-(4-méthylpipérazin-1-yl)-éthyl)amino)phényl)-5-(trifluorométhyl)pyridin-2(1H)-one (composé 12) ;
la 1-(2-chloro-4-((2-(4-(2-hydroxyéthyl)-pipérazin-1-yl)éthyl)amino)phényl)-5-(trifluorométhyl)-pyridin-2(1H)-one (composé 13).

5. Composé selon l'une quelconque des revendications 1 à 4, pour une utilisation comme médicament anti-fibrose.
